**Europäisches Patentamt**

**European Patent Office**

**Office européen des brevets**

(19)

(11) Veröffentlichungsnummer: **0 093 784**
**B1**

(12) **EUROPÄISCHE PATENTSCHRIFT**

(45) Veröffentlichungstag der Patentschrift:
05.08.87

(51) Int. Cl.⁴: **C 11 D 7/42,** C 11 D 3/386 //
G02C13/00, G02C7/04

(21) Anmeldenummer: **82103464.2**

(22) Anmeldetag: **23.04.82**

(54) Enzymatisches Reinigungsmittel für Kontaktlinsen mit pH-kontrollierter Wirkung.

(43) Veröffentlichungstag der Anmeldung:
**16.11.83 Patentblatt 83/46**

(45) Bekanntmachung des Hinweises auf die Patenterteilung:
**05.08.87 Patentblatt 87/32**

(84) Benannte Vertragsstaaten:
**AT BE CH DE FR GB IT LI LU NL SE**

(56) Entgegenhaltungen:
**EP - A - 0 005 131**
**DE - A - 2 854 278**
**FR - A - 2 226 677**
**GB - A - 1 527 010**
**US - A - 3 855 142**

**CHEMICAL ABSTRACTS, Band 97, Nr. 10, 6. September 1982, Seite 424, Nr. 78933d, Columbus, Ohio, USA**
**CHEMICAL ABSTRACTS, Band 90, Nr. 12, 19. März 1979, Seite 88, Nr. 89130w, Columbus, Ohio, USA**

(73) Patentinhaber: **Dr. Thilo & Co. GmbH,**
**Rudolf-Diesel-Ring 21, D-8029 Sauerlach (DE)**

(72) Erfinder: **Kreiner, Christine, Dr., Krenklstrasse 10,**
**D-8000 München 81 (DE)**

(74) Vertreter: **von Füner, Alexander, Dr. et al, Patentanwälte**
**v. Füner, Ebbinghaus, Finck Mariahilfplatz 2 & 3,**
**D-8000 München 90 (DE)**

## Beschreibung

Stand der Technik

Enzymatische Reinigungsmittel für Kontaktlinsen sind bekannt. Die Anwendung enzymatischer Reinigungsmittel liegt nahe, da die Kontaktlinse während des Tragens mit Bestandteilen des Tränenfilms in Berührung kommt und dabei Ablagerungen ausbildet. Der Tränenfilm seinerseits ist komplex aufgebaut. Er besteht aus 3 Schichten, wovon die unterste Schicht aus neutralen Mucopolysacchariden besteht, die die Aufgabe zu erfüllen haben, das hydrophobe Epithel der Cornea benetzbar für die wässerige Phase des Tränenfilms zu machen. Dies ist eine wichtige Voraussetzung für die optische Klarheit der Cornea. Die mittlere Phase des Tränenfilms wird durch eine wässerige Schicht gebildet. Diese wässerige Schicht enthält eine Reihe anorganischer Salze, vorwiegend Natriumchlorid, sowie organische Substanzen wie Glukose, Harnstoff, neutrale und saure Mucopolysaccharide in nach aussen abnehmender Konzentration, Enzyme wie Lysozym und verschiedene Proteine wie Albumin (Gemisch aus Serumalbumin und Specific-Tear-Albumin) und verschiedene Globuline. An der Oberfläche des Tränenfilms – nach aussen hin – befindet sich schliesslich eine dritte Schicht, die Lipidschicht, die den Tränenfilm und damit die Cornea vor dem Austrocknen infolge Verdunsten schützt und verglichen werden kann mit einer hauchdünnen und deshalb optisch transparenten Ölschicht, die auf dem wässerigen Teil des Tränenfilms schwimmt und diesen so nach aussen abschliesst. Die Lipidschicht besteht vorwiegend aus Cholesterylester, Triglyceriden und untergeordnet Phospholipiden.

Die organischen hochmolekularen Bestandteile des Tränenfilms zeigen eine hohe Affinität zu den meisten Kontaktlinsenmaterialien. Man findet somit Protein-, Mucin- und Lipidablagerungen. Je nach chemischen Eigenschaften des entsprechenden Kontaktlinsenmaterials überwiegen die einen oder anderen Ablagerungen. Das grösste Problem innerhalb der Kontaktlinsen stellen hydrophile weiche Kontaktlinsen aus HEMA (Hydroxyäthylmethacrylat) bezüglich der Verschmutzungstendenz während des Tragens dar. HEMA weist eine sehr starke Affinität zu den Proteinen des Tränenfilms auf und wird bei nicht ausreichender Reinigung sehr bald unbrauchbar.

Die enzymatischen Reinigungsmittel für Kontaktlinsen, insbesondere für weiche Kontaktlinsen, bestehen entweder aus reinen Proteasen, wobei diese Produkte vorwiegend Papainprodukte sind (siehe DE-A-2 417 844, US-A-4 285 738), oder aus Kombinationspräparaten, die aus Proteasen, Esterasen bzw. Carboxylester-Hydrolasen sowie Glykosidasen bestehen (siehe D.J. Lloyd, The Ophthalmic Optician, Nov. 10, 1979, 833–839).

In diesen Gemischen wird üblicherweise als Protease Trypisin, als lipidspaltende Carboxylester-Hydrolase Lipase sowie als Glykosidase Amylase eingesetzt. Es gibt ausserdem noch Gemische auf dem Markt, die anstelle der Lipase eine Esterase enthalten (siehe Fries, H. Keller, Enzyme in der Kontaktlinsenpflege, Nov. 3, 1981).

Dies ist im Fall von Lipidablagerungen auf Kontaktlinsen nicht sinnvoll, da nur Carboxylester-Hydrolasen, wie die Lipase, eine Spezifität aufweisen für Glyzerinester sowie eine ausschliesslich auf emulgierte Glyzerinester gerichtete Aktivität besitzen, somit nur bei nichtgelösten Lipiden reagieren. Andererseits besitzt Lipase keine strenge Substratspezifität. Es werden nämlich Ester von Fettsäuren verschiedenster Kettenlänge, gesättigt wie ungesättigt, hydrolysiert.

Liegt ein solches Gemisch vor aus Trypsin, Lipase und α-Amylase, so würde der Zusatz eines Netzmittels einen Kunstfehler darstellen, da die Lipase durch ein Netzmittel einen Aktivitätsverlust erleidet, entsprechend ihrer speziellen Affinität für Fett-Wasser-Grenzflächen.

Die derzeit bekannten enzymatischen Reinigungsmittel weisen folgende Nachteile auf:

1. Die Enzyme erfahren eine schnelle Inaktivierung in wässeriger Lösung. Dies führt zu einem starken Wirkungsverlust bei der praktischen Handhabung des Enzymreinigers wie Auflösen eines Pulvers oder von Tabletten in Wasser. Diese Inaktivierung erfolgt sowohl durch Autolyse der Proteasen als auch, falls Gemische verwendet werden, durch proteolytischen Abbau der im Gemisch verwendeten anderen Enzyme.

2. Alle Enzyme stellen artfremde Antigene dar. Dies bedeutet, dass Enzymrückstände auf den Kontaktlinsen eine allergische Proteinkonjunktivität erzeugen können. Dies ist vor allem im Zusammenhang mit weichen Kontaktlinsen von Bedeutung, die aufgrund ihrer hohen Affinität zu Proteinen auch die als Reiniger verwendeten Enzyme binden können, so dass bei unsachgemässer Handhabung durch den Kontaktlinsenträger diese Enzymrückstände mit dem Auge in Berührung kommen können.

Daneben sind einige Enzyme, wie z.B. Chymotrypsin, toxisch am Auge.

Aufgabenstellung

Die Aufgabenstellung bestand darin, die oben erwähnten Nachteile zu beseitigen, ohne die Vorteile zu beeinträchtigen

Problemlösung:

Man musste also erfindungsgemäss einen enzymatischen Reiniger im obigen Sinn für Kontaktlinsen so modifizieren, dass zunächst während des Reinigungsvorganges die Autolyse der Protease sowie die Proteolyse der anderen, im Gemisch beteiligten Enzyme unterdrückt wird, sodann nach erfolgter Spaltung der Ablagerungen auf der Kontaktlinse eine rasche Selbstzersetzung der Enzyme eintritt, wodurch gutlösliche und damit leicht zu entfernende niedermolekulare Bruchstücke entstehen, so dass die Gefahr einer möglichen Proteinallergie auf noch an der Kontaktlinse anhaftende Enzymrückstände nicht mehr besteht.

Gegenstand der Erfindung ist also eine Brausetablette mit folgenden Bestandteilen:

|  | bezogen auf die Lösung | bezogen auf die Tablette |
|---|---|---|
| Trypsin | 0,5 bis 5,0% | 5,0 bis 50 % |
| α-Amylase | 0,01 bis 0,5% | 0,1 bis 5,0% |
| Lipase | 0,01 bis 0,5% | 0,1 bis 5,0% |
| Citronensäure·H$_2$O | 0,20 bis 2,1% | 3,0 bis 31 % |
| NaHCO$_3$ | 0,55 bis 4,1% | 8,1 bis 61 % |
| Ca-Acetat | 0,001 bis 0,9% | 0,01 bis 9,0% |
| EDTA | 0,005 bis 2,0% | 0,05 bis 20 % |

Im einzelnen lässt sich die Erfindung wie folgt erläutern: Löst man ein Gemisch aus einer Protease, einer Carboxylesterhydrolase und einer Glykosidase mit einer genau dosierten Menge an Citronensäure und Natriumhydrogencarbonat in Wasser, so entwickelt sich CO$_2$, das durch Entweichen aus der Lösung zu einer langsamen, kontinuierlichen pH-Verschiebung vom schwach sauren bzw. neutralen Milieu in das alkalische Milieu führt. Bei schwach saurem bis nautralem pH ist die proteolytische Wirkung von Proteasen, insbesondere Trypsin, reduziert. Ihr Wirkungsoptimum liegt bei einem pH von 8 bis 9. Dadurch, dass der pH-Wert, der durch Auflösen des oben benannten Enzymgemisches in Wasser entstandenen Lösung zunächst schwach sauer bis neutral ist, kann die Protease somit noch nicht ihre volle Wirkung entfalten. Dies bedeutet, dass die Protease bei diesem pH-Wert nicht die anderen in der Lösung vorliegenden Enzyme inaktiviert. Diese beiden Enzyme bleiben somit bei diesem pH-Wert in Lösung stabil und können ihre katalytische Wirkung voll entfalten und die auf der Kontaktlinse vorhandenen Ablagerungen enzymatisch abbauen. Durch das langsame Entweichen von CO$_2$ verschiebt sich der pH-Wert zum schwach alkalischen. Dadurch nimmt die proteolytische Wirksamkeit der Protease zu. Die Protease greift nun nicht nur die beiden im Gemisch befindlichen Enzyme an unter Proteolyse, sondern autolysiert sich selbst. Die Konzentration an intakten Enzymen in der Lösung sinkt mit zunehmendem pH-Wert, bis nach ca. 6 bis 8 Stunden keine Enzymkonzentrationen an der Linse mehr festzustellen sind, die am Auge toxisch reagieren könnten.

Diese Kombination bewirkt somit, dass zunächst die volle reinigende Wirkung der eingesetzten Enzyme für eine ausreichende Zeit eintritt. Durch eine genau bemessene Menge an Citronensäure und Natriumhydrogencarbonat verschiebt sich jedoch der pH-Wert in der Lösung so, dass die Stabilität der Enzyme abnimmt, so dass eine schnelle Zersetzung der Enzyme eintritt.

Wirkung der Kontrolle anhand eines Anwendungsbeispiels

a) Enzymaktivität in Abhängigkeit von der Zeit:

Es wurde die Trypsinaktivität eines Gemisches A mit dem eines Gemisches B) verglichen. Das Gemisch A) enthielt im Gegensatz zu B) Citronensäure und Natriumhydrogencarbonat.

| Gemisch A): | bezogen auf die Lösung | bezogen auf die Tablette, pH = 6,2 |
|---|---|---|
| Trypsin | 1,80 % | 26,6% |
| α-Amylase | 0,13 % | 1,9% |
| Lipase | 0,13 % | 1,9% |
| Citronensäure·H$_2$O | 1,04 % | 15,4% |
| NaHCO$_3$ | 2,10 % | 31,1% |
| Ca-Acetat | 0,49 % | 7,3% |
| EDTA | 1,00 % | 14,8% |
| PEG 6000 | 0,06 % ⎤ | 0,9% ⎤ |
|  |    ⎬ 0,07% |    ⎬ 1,0% |
| Silikonöl | 0,006% ⎦ | 0,1% ⎦ |

| Gemisch B): | bezogen auf die Lösung, ph = 7,35 |
|---|---|
| Enzyme wie in A) |  |
| Citronensäure·H$_2$O | 0,20% |
| Na$_2$HPO$_4$ | 3,20% |
| CaCl$_2$ | 0,06% |
| EDTA | 0,20% |
| PEG 4000 | 96,36% |

Während im Gemisch B) die Trypsinaktivität kontinuierlich in Abhängigkeit der Zeit absank, wurde bei dem Gemisch A) eine langsame Steigerung der Trypsinaktivität festgestellt, mit einem Wirkungsoptimum nach 3 bis 4 Stunden, danach sank die Trypsinaktivität kontinuierlich ab. Die Amylase-Aktivität sank im Gemisch A) nach 4 Stunden um ca. 25%, im Gemisch B) .(ohne pH-Verschiebung) waren nach 4 Stunden noch 91% der ursprünglichen Aktivität vorhanden. Nach 6 Stunden war im Gemisch A) nur noch ca. die Hälfte der ursprünglichen Enzymaktivität vorhanden, während im Gemisch B) noch 88% Amylase-Aktivität nachweisbar war. Dies zeigt, dass durch die pH-Verschiebung die proteolytische Wirkung von Trypsin nach 3 bis 4 Stunden einsetzt, so dass in den ersten 3 Stunden zwar zunächst die reinigende Wirkung der Amylase zum Tragen kommt, danach aber die Amylase schnell inaktiviert wird.

Die Lipase-Aktivität sank in analoger Weise wie bei Amylase beschrieben.

b) Reinigungswirkung in Abhängigkeit vom pH-Wert:

Zur Überprüfung der Reinigungswirkung der Enzymgemische A) und B) wurden auf weiche HEMA-Linsen Niederschläge erzeugt, die in ihrer Morphologie bei Betrachtung mit dem Rasterelektronenmikroskop den natürlich entstandenen Ablagerungen gleichen. Dazu wurden die Linsen einzeln in kleinen Gläschen mit künstlichem Tränenfilm überschichtet und bei 37 °C im Trockenschrank solange gehalten, bis die Flüssigkeit nahezu eingetrocknet war. Danach wurden die Linsen mit physiologischer Kochsalzlösung abgespült und erneut mit Tränenfilm beschichtet und im Trockenschrank die Lösung eingetrocknet. Nach 10 Beschichtungsvorgängen wurden die Linsen vor dem Abspülen mit physiologischer Kochsalzlösung für $\frac{1}{4}$ Stunde unter UV-Licht gelegt, sodann wurde der Beschichtungsvorgang bei 37 °C solange wiederholt, bis ein mit physiologischer Kochsalzlösung nicht mehr abspülbarer Niederschlag entstanden war. Diese Linsen wurden dann für Reinigungsversuche verwendet.

Zusammensetzung des künstlichen Tränenfilms

Die Bestandteile werden in 100 ml destilliertem Wasser gelöst unter leichtem Erwärmen und Rühren.

| | | |
|---|---|---|
| Mucin | 0,04 | g |
| Albumin | 0,18 | g |
| $\alpha$-Globulin | 0,08 | g |
| $\beta$-Globulin | 0,08 | g |
| $\gamma$-Globulin | 0,08 | g |
| Lysozym | 0,18 | g |
| Harnstoff | 0,035 | g |
| Glucose | 0,0025 | g |
| Natriumchlorid | 0,55 | g |
| Calciumchlorid | 0,013 | g |
| Tripalm. Glycerid | 0,01 | g |
| Cholesteryloleat | 0,01 | g |
| Cholesterylpalmitat | 0,01 | g |
| $Na_2HPO_4$ | 0,250 | g |
| $NaH_2PO_4$ | 0,195 | g |
| $NaHCO_3$ | 0,218 | g |

Die Reinigungsversuche wurden in der Weise durchgeführt, dass in jeweils 5 ml der durch Auflösen des Gemisches A) bzw. des Gemisches B) entstandenen Lösungen jeweils eine Linse eingelegt wurde und die Reinigungswirkung nach 4 Stunden durch mikroskopische Untersuchung der Oberfläche der Linsen überprüft wurde. Es wurden für beide Gemische 30 Linsen verwendet. Von den mit dem Enzymgemisch A) gereinigten 30 Linsen waren 24 Linsen sauber (= 80%), 4 Linsen wiesen noch schwache Ablagerungen auf (= 13,3%), 2 Linsen waren unverändert mit Ablagerungen versehen (= 6,6). Von den mit dem Enzymgemisch B) gereinigten 30 Linsen waren 11 Linsen sauber (36,6%), 12 Linsen wiesen noch schwache Ablagerungen auf (40%), 7 Linsen waren mit unveränderten Ablagerungen versehen (23%).

Dieses Ergebnis ist so zu interpretieren, dass der Hauptanteil an Ablagerungen auf den künstlich beschichteten HEMA-Linsen aus Proteinen bestand, so dass durch die aufgrund der pH-Verschiebung eintretende Steigerung der proteolytischen Eigenschaften des Gemisches A) ein stärkerer Reinigungseffekt zu erzielen ist als bei dem Gemisch B), wo der pH-Wert konstant blieb.

Es ist anzunehmen, dass bei reinen Lipidablagerungen der Unterschied in der Reinigungswirkung zwischen A) und B) nur gering ist.

## Patentanspruch

Brausetablette auf der Basis von Trypsin, $\alpha$-Amylase sowie Lipase und Citronensäure, dadurch gekennzeichnet, dass sie neben 5 bis 50% Trypsin, 0,1 bis 5,0% $\alpha$-Amylase, 0,1 bis 5,0% Lipase, 3,0 bis 31% Citronensäure, 8,1 bis 61% $NaHCO_3$, 0,01 bis 9,0% Ca-Acetat und 0,05 bis 20% EDTA, bezogen auf die Brausetablette, enthält.

## Claim

An effervescent tablet based on trypsin, $\alpha$-amylase, lipase and citric acid, characterized in that it contains, relative to the effervescent tablet, 5 to 50% trypsin, 0.1 to 5.0% $\alpha$-amylase, 0.1 to 5.0% lipase, 3.0 to 31% citric acid, 8.1 to 61% $NaHCO_3$, 0.01 to 9.0% calcium acetate and 0.05 to 20% EDTA.

## Revendication

Tablette effervescente à base de trypsine, d'$\alpha$-amylase ainsi que de lipase et d'acide citrique, caractérisée par le fait qu'elle comprend outre 5 à 50% de trypsine, 0,1 à 5,0% d'$\alpha$-amylase, 0,1 à 5,0% de lipase et 3,0 à 31% d'acide citrique, de 8,1 à 61% de $NaHCO_3$, de 0,01 à 9,0% d'acétate de calcium et de 0,05 à 20% d'AEDT (acide éthylènediamine tétraacétique) par rapport à la tablette effervescente.